# EUROPÄISCHE PATENTANMELDUNG

(11) **EP 1 208 871 A2**
(43) Veröffentlichungstag der Anmeldung: **29.05.2002**
(21) Anmeldenummer: 01250410.6
(22) Anmeldetag: 22.11.2001
(51) Int. Cl.: A61M 36/00, A61M 37/00

(54) **Verfahren und Applikator zum Positionieren und/oder Auswerfen von Strahlenquellen über Hohlnadeln in biologische Gewebe**

(30) Priorität: 22.11.2000 DE 10058163
(71) Anmelder: jojumarie Intelligente Instrumente GmbH, 13125 Berlin (DE)
(72) Erfinder: Lüth, Tim, Prof. Dr., 13156 Berlin (DE)
(74) Vertreter: Hengelhaupt, Jürgen D., Dipl.-Ing.

(57) **Zusammenfassung**

Die Erfindung betrifft ein Verfahren dadurch gekennzeichnet ist, dass unterschiedliche Seeds (4) nach Isotopentyp (Material, Dosis, Halbwertzeit) getrennt in Magazinen (8) enthalten sind, der Hohlnadel (2) derart zugeführt werden, dass das Wechseln der einzelnen Magazine (8) mittels eines Magazinwechslers (9) erfolgt, der in einer Wechslerführung (16) vertikal zur Injektionsrichtung der Seeds (4) geführt wird, wobei die Lage (Position und Orientierung) der Nadel (2) relativ zu einem Lagereferenzkörper (13) gemessen wird und eine optimale Seedablageposition (23) akustisch und/oder visuell und/oder taktil erfasst wird, wobei die Auswahl eines Magazins (8) und eines auszuwerfenden Seeds (4) aus dem Magazin (8) aus der aktuellen Position der Nadel (2) im Gewebe (3) und aus einem in Listenform mit Angabe des Seed-Typs und der x-y-z-Koordinaten relativ zu einem Lagereferenzkörper (13) erstellten Behandlungsplan für eine Dosisverteilung abgeleitet wird.

## Beschreibung

Die Erfindung betrifft ein Verfahren und einen Applikator zum Positionieren von Strahlenquellen über Hohlnadeln zur Erzielung einer optimalen Dosisverteilung beim Einbringen von unterschiedlichen Strahlenquellen in biologisches Gewebe gemäß den Oberbegriffen der Ansprüche 1 und 12.

Das Verfahren und der Applikator sind insbesondere für die Behandlung von Prostatakrebs mit schwachradioaktiven Strahlenquellen (Seeds) geeignet.

In der Brachytherapie wird Tumorgewebe aus möglichst kurzer Entfernung bestrahlt. Dies geschieht beispielsweise mit kleinen radioaktiven bzw. gamma-strahlenden Quellen, die über Hohlnadeln in das erkrankte Gewebe eingestochen werden. Ein Tumor wird dabei typischerweise mit mehreren Hohlnadeln gespickt. Dabei lassen sich zwei Formen unterscheiden:
1) Die Quellen strahlen mit starker Dosis. Sie werden nur kurze Zeit in die Nadeln eingeführt und dann wieder herausgezogen. Die Strahlung wird durch die Wand der Hohlnadeln hindurch abgegeben. Die Behandlung wird gegebenenfalls wiederholt. Die Quellen haben eine sehr große Halbwertszeit in der Größenordnung von Jahren.
2) Die Quellen strahlen mit niedriger Dosis. Sie sind saatkorngroß (Seeds) und werden durch die Hohlnadeln in das Gewebe ausgestoßen bzw. implantiert. Sie verbleiben in dem Gewebe. Die Halbwertszeit beträgt Wochen bzw. Monate und sinkt in diesem Zeitraum auch unter eine therapeutische oder schädigende Wirkung. Für die Behandlung werden beispielsweise schwachradioaktive Isotope der Elemente Jod, Kobalt oder Palladium eingesetzt.

Für den therapeutischen Effekt von grundlegender Bedeutung sind dabei folgende Kriterion: Für die Behandlung mit schwachradioaktiven Elementen ist eine möglichst gleichmäßige Dosisverteilung im erkrankten Gewebe (z.B. Prostata) auf hohem Niveau erforderlich. Das gesunde Gewebe (z.B. Harnleiter, Samenbläschen) darf nicht über eine maximale Dosis hinaus bestrahlt werden, um erneute oder verschleppte Gewebeerkrankungen auszulösen.

Mit den gegenwärtig bekannten Systemen wird die Plazierung einer Menge gleichartiger Seeds mit gleicher Strahlendosis und Halbwertszeit unterstützt.

Auf der Grundlage des therapeutischen Effektkriteriums bestimmt eine Software zur Dosisplanung die Positionen der Seeds in einem kartesischen Koordinatensystem relativ zu einer extrakorporal befestigten Lochrasterplatte. Das Lochraster hat typischerweise einen Lochabstand von 5 mm. Die Nadeln für die Seedimplantierung werden nach den Vorgaben der Dosisplanung durch das Lochraster geführt. Sie werden durch die Haut und gesundes Gewebe hindurch in das Tumorgewebe eingestochen. Als Tiefenkontrolle dient entweder ein Maßstab, der auf den Nadeln aufgebracht ist, oder ein mechanischer Maßstab zusätzlich zur Nadel.

Es gibt gegenwärtig zwei grundlegende Verfahren für das Einbringen der Seeds:
1) Das Einstechen von Hohlnadeln, die bis zur Nadelspitze mit einer Seedkette (RAPID STRAND/TM) aus gleichartigen therapeutischen Seeds vorgeladen sind. Die Seedkette wird dann an der Nadelspitze im Gewebe verankert und die Nadeln zurückgezogen. Dabei verbleibt die Seedkette im Gewebe.
   Es sind zwei Verfahren für die Herstellung und Benutzung von Seedketten bekannt.
   Aus der US 5460592 (Langton et al.) ist eine Vorrichtung zur Herstellung und Transport von Seeds bokannt, die in ein bio-absorbierbares Material eingebettet sind. Die Ketten werden nach der Eingriffsplanung einem Transportbehälter entnommen und auf die erforderliche Länge gekürzt, bevor sie in Hohlnadeln eingeführt werden.
   In der US 5938583 (Grimm) ist eine besonders einfach zu befüllende und zurückziehbare Hohlnadel beschrieben.
   In der US 6010446 ist ein bio-absorbierbarer Abstandshalter zwischen den Seeds beschrieben, der eine zeitnahe Herstellung von Seedketten erlaubt.
   Aus einer Produktbeschreibung (RADIOACTIVE SEED SHUTTLE) der Firma MED-TEC (PO Box 320 Orange City, Iowa 51041, USA) ist eine entsprechendes Produkt bekannt, dessen Funktionsweise jedoch nicht beschrieben ist.
   Weiterhin ist aus Produktbeschreibungen (EXPRESS SEEDING CARTRIDGE) aus dem Jahr 1999 der Firma Indigo Medical Inc. (10123 Alliance Road, Cincinanati, Ohio, 45242) eine vorgeladene Seed-Cartridge zum direkten Befüllen einer Nadel bekannt.
2) Das Einstechen von Hohlnadeln, durch die später einzelne Seeds (Free-seeds) an der Nadelspitze ausgeworfen werden. Die Einzelseeds werden dabei einzeln und manuell durch einen Apparat an der Nadelspitze ausgeworfen. Die Nadel wird anschließend über eine mechanische Raste manuell entsprechend dem geplanten Seedabstand im Planungsraster zurückgezogen bevor das nächste Einzelseed ausgeworfen wird. Die Einzelseeds werden über ein federbasiertes Magazin nachgeladen. Ein solcher Apparat ist aus der US 5860909 bekannt.

In der US 6102844 ist eine Lösung beschrieben, nach welcher der manuelle Auswurf der Seeds faseroptisch überwacht wird.

Dabei wird im Gegensatz zu dem Verfahren aus US 5860909 ein mechanisch sehr aufwendiges Revolvermagazin verwendet.

Aus der WO 00/61229 A1 ist es bekannt, für die Behandlung auch unterschiedliche Seeds bzw. Seeds-Mixturen zu entnehmen. Die Lage der Nadel relativ zu einem Lagereferenzkörper wird dabei visuell dargestellt, wobei auch eine optimale Seedablageposition erfasst wird. Die Auswahl eines Magazins und eines auszuwerfenden Seeds aus dem Revolvermagazin wird aus der aktuellen Position der Nadel im Gewebe bestimmt.

Aus der US 5,906,574 A kann entnommen werden, für eine optimierte Behandlung die Verbindung zum Behandlungsplan herzustellen.

Die Seedanlieferung geschieht gegenwärtig pünktlich zu bestimmten Behandlungstagen auf Bestellung des behandelnden Arztes für die jeweiligen Patienten. Die Bestellung beruht auf einer groben Berechnung des Seed-Bedarfs für den Patienten. Die Berechnung beruht auf der Abschätzung des Prostatavolumens über medizinische Bilddaten, die typischerweise Wochen vor dem eigentlichen Eingriff liegen. Bis zum Behandlungstag kann sich die Größe der Prostata jedoch noch verändern.

In der Praxis gibt es ein erhebliches Risiko, an diesem Tag zuwenig gleichartige Seeds (Dosis, Halbwertszeit) für den Patienten vorgehalten zu haben. Fehlen Seeds, dann muss die Behandlung abgebrochen werden. Leider läßt sich dies erst feststellen, wenn sich der Patient bereits in Narkose befindet und die Prostata mit einer Ultraschallsonde neu vermessen wurde und die zeitnahe Dosisplanung durchgeführt worden ist.

Die Seeds sind teuer, lassen sich nicht zurückgeben und gegenwärtig auch nicht für andere Patienten zu späteren Behandlungstagen einsetzen. Sollten zuwenig Seeds vorhanden sein, verfallen alle Seeds, da die OP nicht durchgeführt werden kann. Sollten zuviele Seeds vorhanden sein, verfallen alle nichtplazierten Seeds, da diese anschließend nicht weiter verwendet werden können. Dies ist vor allem bei den sehr teuren Palladium Seeds mit kurzer Halbwertszeit von knapp 3 Wochen ein erheblicher Schaden. Die fehlende Möglichkeit der Verwendung unterschiedlicher Dosiswerte ist daher ein finanzieller Nachteil für Patient und Arzt bzw. ein erhebliches Behandlungsrisiko für den Patienten.

Durch die Verwenden von ausschließlich gleichartigen Seeds (Dosis, Halbwertszeit) kann eine optimale Dosisverteilung in Gewebe nicht erreicht werden. Vor allem die immer bessere Früherkennung und die verbesserte Bildgebung würden eine angepasstere Dosisverteilung erfordern. Diese könnte auf Gewebeeigenschaften Rücksicht nehmen, um beispielsweise das Risiko der Inkontinenz oder der Unfruchtbarkeit zu reduzieren.

Mit den bekannten Vorrichtungen wäre ein Mischen von Strahlenquellen zwar prinzipiell bei sehr hohem manuellen Einsatz möglich, jedoch mit einem unverantwortlichen hohem Risiko für den Patienten verbunden.

Alle Systeme sind für den reinen Handbetriebs ausgelegt. Sie lassen sich nur aufwendig für die Automatisierung des Mischens unterschiedlicher Seeds umrüsten.

Alle Systeme unterstützen nur das Verfahren der Einzelseeds bzw. nur das Verfahren der Seed-Ketten. Ein gleichzeitiger Einsatz beider Verfahren bei einem Patienten ist nicht mit vertretbarem Aufwand möglich.

Das patientenspezifische manuelle Mischen und Befüllen von Magazinen und Hohlnadeln ist mit einem hohen Gesundheitsrisiko für das medizinsch-technische Personal verbunden. Es kann zu Kommunikationsfehlern zwischen Verantwortlichen für die Dosisplanung, den Befüllern und den Implantologen kommen. Damit ist auch ein Risiko der fehlerhaften Seed-Implantierung und einer fehlerhaften Behandlung verbunden.

Das Vorladen der Nadeln hat gegenüber den Einzelseeds den Verlust an Flexibilität bei der Planung zur Folge, wenn die Seeds nicht exakt wie vorgeplant in Tumorgewebe abgelegt werden oder abgelegt werden können. Wandern Seeds nach der Ablage oder kommt es zu einer fehlerhaften Bedienung sind die vorgeladenen Nadeln von Nachteil für die Behandlungsqualität.

Aufgabe der Erfindung ist es, die bekannten Nachteile des Standes der Technik zu vermeiden und ein Verfahren und einen Applikator zu entwickeln, mit denen das Implantieren von unterschiedlichen Seeds (Material, Dosis, Halbwertszeit) sowie von Abstandshaltern in Tumorgewebe bzw. das Positionieren von Seeds, Abstandshaltern und Stoppern in Hohlnadeln oder in Vorrichtungen für die Seedkettenherstellung unterstützt wird, um nach der Seed-Implantierung eine optimale Dosisverteilung im Tumorgewebe erreichen zu können.

Diese Aufgabe wird durch ein Verfahren nach den Merkmalen des Anspruchs 1 und durch eine Vorrichtung nach den Merkmalen des Anspruchs 12 gelöst.

Das Verfahren zum Positionieren von Strahlenquellen (Seeds), Abstandshaltern und Stoppern in Hohlnadeln ist dadurch gekennzeichnet, dass unterschiedliche Seeds, die nach Isotopentyp (Material, Dosis, Halbwertzeit) getrennt in Magazinen enthalten sind, der Hohlnadel derart zugeführt werden, dass das Wechseln der einzelnen Magazine mittels eines Magazinwechslers erfolgt, der in einer Wechslerführung vertikal zur Injektionsrichtung der Seeds geführt wird, wobei die Lage (Position und Orientierung) der Nadel relativ zu einem Lagereferenzkörper gemessen wird und eine optimale Seedablageposition akustisch und/oder visuell und/oder taktil erfasst wird, wobei die Auswahl eines Magazins und eines auszuwerfenden Seeds aus dem Magazin aus der aktuellen Position der Nadel im Gewebe und aus einem in Listenform mit Angabe des Seed-Typs und der x-y-z-Koordinaten relativ zu einem Lagereferenzkörper erstellten Behandlungsplan für eine Dosisverteilung abgeleitet wird.

Der Applikator zum Positionieren von Strahlenquellen (Seeds), Abstandshaltern und Stoppern in Hohlnadeln und zum Auswerfen derselben aus Hohlnadeln ist dadurch gekennzeichnet, dass eine Magazinwechsleraufnahme mit einem in einer Wechslerführung angeordneten, vertikal zur Injektionsrichtung des Seeds führbaren Magazinwechsler mit Magazinen, die verschiedene Seeds, jeweils getrennt nach Isotopentyp (Material, Dosis, Halbwertszeit), enthalten, eine Nadelaufnahme für eine Hohlnadel aufweist, an die über einen Schnellverschluss (18) die Magazinwechsleraufnahme angeflanscht ist, wobei die Nadel Seeds aus einem der Magazine aufnehmen und über eine Auswurfeinrichtung wie Auswurfdraht oder Auswurfstilett ausgeben kann, wobei die Magazinwechsleraufnahme mit einem Schlitten mit einer Gleitführung zur reproduzierbaren, genauen Positionierung der Nadel verbunden ist.

Das Verfahren und der Applikator nach der Erfindung sind insbesondere für die Behandlung von Prostatakrebs mit schwachradioaktiven Strahlenquellen (Seeds) vorteilhaft anwendbar.

Das Mischen von Seeds unterschiedlichen Typs, z.B. Jod, Palladium mit unterschiedlicher Halbwertszeit und Dosis wird möglich. Damit lassen sich verbliebene Seeds vorheriger Eingriffe gegebenenfalls noch verwenden. Dieses wirkt kostensenkend. Das Risiko für Behandlungsabbrüche vor oder nach Einleitung der Narkose durch fehlende Seeds sinkt.

Es ist möglich, Strahlenquellen unterschiedlicher Halbwertszeiten und unterschiedlicher Strahlendosis zu verwenden. Damit lassen sich einige Geweberegionen kurz und hart, andere länger und weich bestrahlen, je nachdem welche Gewebetherapierung sinnvoll ist. Durch das freie Mischen von Seeds kann eine optimale Dosisverteilung im Gewebe geplant und erreicht werden. Nebenwirkungen wie Tumorneubildung oder Inkontinenz sinken bei Behandlung von Prostatakrebs.

Die Dosisplanung kann aktuell und nach jedem Seedauswurf korrigiert und angepasst werden.

Es können sowohl Einzel-Seeds in Hohlnadeln zum Einsatz kommen als auch Hohlnadeln zeitnah mit verschiedenen Seeds befüllt werden. Da die Dosisplanung ständig aktualisiert wird, wird ein besseres Behandlungsergebnis erzielt. Eine entsprechende Planung der Dosisverteilung mit dem neuen System kann sehr einfach realisiert werden.

Die Seeds können mit Stopper-Seeds (Nadelstopfen) und Spacer-Seeds (Abstandshalter) gemischt werden. Dies hat den Vorteil, mit einer Nadel auch größere Zwischenräume ohne Seeds und Abstandshalter zu realisieren.

Der Applikator läßt sich mit sehr geringem Aufwand vollständig automatisieren.

Das automatisierte Ausstoßen der Seeds an vorgeplanten Positionen, das Mitprotokollieren und die Rückkopplung in die Planung reduzieren das Risiko einer Fehlbehandlung und erhöht die Qualität der Therapie. Die Fehlerquote wird gesenkt, es gibt nachweisbare Behandlungsergebnisse, und die erforderliche Konzentrationsschwelle sinkt.

Des weiteren vereinfacht die Verwendung von Seeds mit unterschiedlicher Dosis und Halbwertszeit auch die Produktion der Seeds, da dann bei der Produktion die tatsächliche Einzelseed-Dosis gemessen werden kann und ein größerer Anteil der Seeds aus der Produktion zum Einsatz beim Patienten gelangen kann.

Die Strahlenbelastung und das Gesundheitsrisiko für das Personal durch das manuelle Befüllen sinken.

Zweckmäßige Ausführungsformen des Verfahrens und des Applikators nach der Erfindung sind in den Unteransprüchen beschrieben.

Nachfolgend wird die Erfindung in einem Ausführungsbeispiel eines Applikators näher beschrieben. In der zugehörigen Zeichnung zeigen:
- Fig. 1 :: eine schematische Darstellung eines Seed-Applikators mit Nadel im Gewebe,
- Fig. 2 :: eine schematische Darstellung einer Magazinwechsleraufnahme des Seed-Applikators nach Fig. 1,
- Fig. 3 :: eine lineare Ausführung des Magazinwechslers nach Fig. 1 und 2,
- Fig. 4 :: eine schematische Darstellung eines positionsgesteuerten Auswurfs von Seeds im Gewebe und
- Fig. 5 :: die Darstelllung einer Befüllung von Nadeln für mehrere Tumorgebiete.

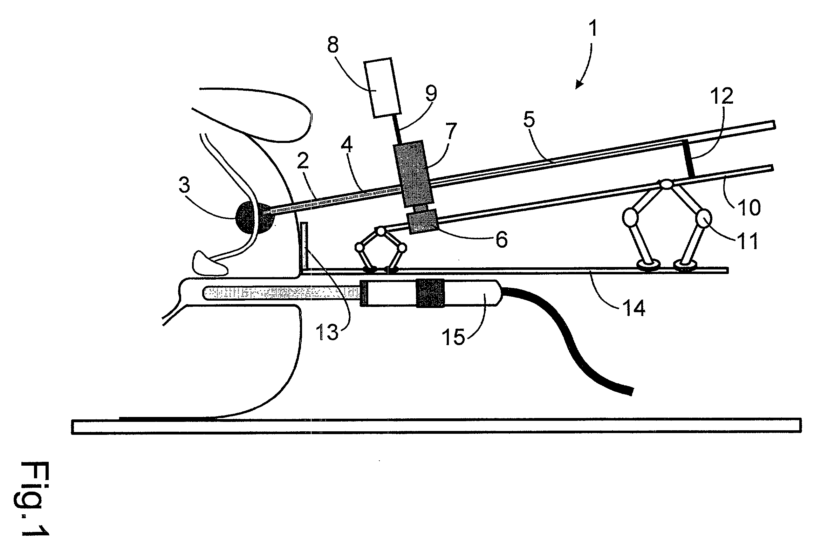

Die Fig. 1 zeigt eine Ausführungsform eines Applikators 1 nach der Erfindung. Danach besteht der Applikator 1 im wesentlichen aus einer Magazinwechsleraufnahme 7 mit einem Magazinwechsler 9 mit Magazinen 8, aus einem Schlitten 6 mit einer Linear- bzw. Gleitführung 10 mit Fixierelementen 11 auf einer Platte 14, aus Lagereferenzkörpern 13, aus einer Nadelaufnahme 17 für eine Hohlnadel 2 mit Auswurfeinrichtung 5 für Seeds 4 mit einer Bremse 12.

In der Fig. 1 ist der Seed-Applikator 1 mit angeflanschter Hohlnadel 2 dargestellt. Die Hohlnadel 2 befindet sich bereits im Prostata-Gewebe 3. Ein Seed 4 wird über die Auswurfeinrichtung 5 aus dem Magazin 8 und durch die Nadel 2 in das Gewebe 3 gedrückt.

Der gesamte Seed-Applikator 1 kann linear in Nadelrichtung aus dem Gewebe 3 herausgezogen werden. Dies geschieht über den Schlitten 6, der an der Magazinwechsleraufnahme 7 des Seed-Applikators 1 angebracht ist. Der Schlitten 6 gleitet auf der Linearführung 10, die über Fixierelemente 11 in jeder gewünschten Lage (Position und Orientierung) relativ zu dem Lagereferenzkörper 13 stabil befestigt werden kann. In der Fig. 1 geschieht dies über Gelenkarme 11, die vorne und hinten die Linearführung 10 auf einer Platte 14 stabilisieren, die fest mit dem Lagereferenzkörper 13 verbunden ist. Die Auswurfeinrichtung 5 kann über die Bremse 12 relativ zu dem Lagereferenzkörper 13 bzw. der Linearführung 10 festgestellt werden.

Der Lagereferenzkörper 13 ist eine bekannte Geometrie, deren relative Lage zu den bildgebenden Systemen und Wegmeßsystemen bekannt ist.

Anstatt der Gelenkarme 11 können auch aktiv positionierende Antriebe zur Ausrichtung der Linearführung 10 verwendet werden. Der Schlitten 6 kann auch über einen Antrieb angetrieben und gebremst werden.

Die Auswurfeinrichtung 5 ist ein drahtförmiges bzw. stilettartiges Instrument und kann auch motorisch angetrieben und gebremst werden. Dies ist erforderlich, wenn die Nadeln 2 im Gewebe 3 vorgeladen werden.

Die Position der Auswurfeinrichtung 5 gegenüber dem Lagereferenzkörper 13 kann über das Fixierelement 11 in der Lage festgestellt werden. Dies ist erforderlich, wenn die vorgeladenen Nadeln 2 aus dem Gewebe 3 herausgezogen werden.

Die Position der Nadel 2 im Gewebe 3 kann über eine Rektalsonde 15 erfasst werden. Die Position des Applikators 1 kann ebenfalls über geometrische Überlegungen aus dem Ultraschallbild berechnet und die Bewegungsbahn der Nadeln 2 abgeleitet werden. Über Wegmeßsysteme auf der Linearführung 10 kann die Bewegung des Applikators 1 gemessen werden.

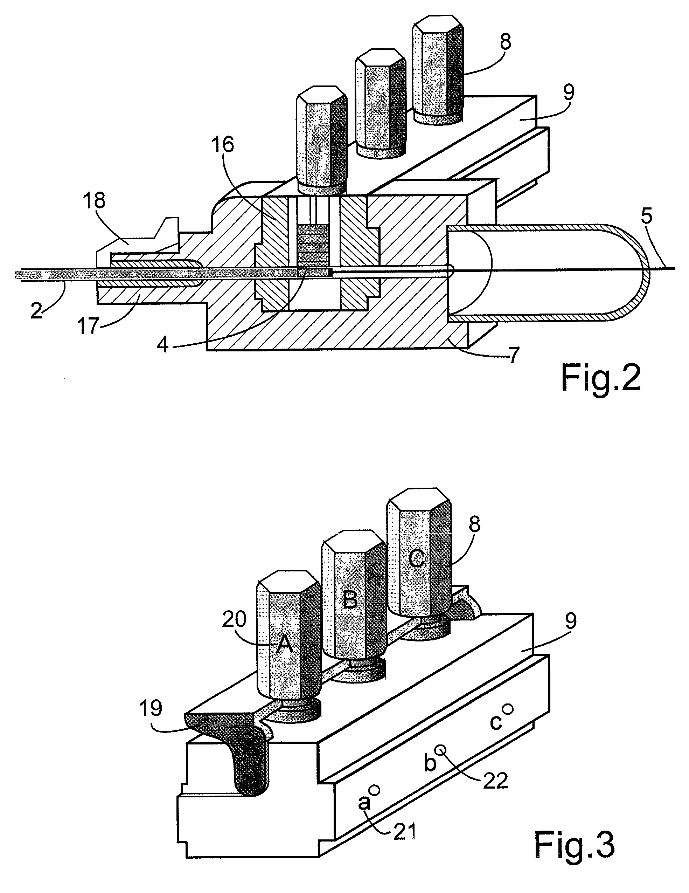

Fig. 2 zeigt die Magazinwechsleraufnahme 7 des Seed-Applikators 1 und einen Magazinwechsler 9 mit Seed-Magazinen 8 im Querschnitt. Die Seed-Magazine 8 stecken in dem Magazinwechsler 9. Der Magazinwechsler 9 gleitet in einer Wechslerführung 16. Die erlaubten Positionen des Magazinwechslers 9 in der Wechslerführung 16 werden über einen Stell- oder Rastmechanismus erreicht. In den erlaubten Positionen kann ein einzelnes Seed 4 über die Auswurfeinrichtung 5 in die Nadel 2 geschoben werden. Die Nadel 2 ist in der Nadelaufnahme 17 über einen Schnellverschluss 18 fixiert. Der Magazinwechsler 9 gleitet in der Linearführung 10 in der Magazinwechsleraufnahme 7 und bringt jeweils ein Magazin 8 in die Position vor der Auswurfeinrichtung 5. Eine Kugelraste sichert die exakte Position des einzelnen Magazins 8 vor der Auswurfeinrichtung 5. Die Auswurfeinrichtung 5 schiebt das Seed 4 aus dem Magazin 8 und an die gewünschte Position in der Nadel 2 bzw. wirft es an der Nadelspitze aus. Die Position der Auswurfeinrichtung 5 relativ zum Nadelreferenzpunkt wird über einen Maßstab erfasst. Die Nadel 2 wird in ihrer Position zum Nadelreferenzpunkt über den Schnellverschluss 18 in ihrer Position gesichert.

Fig. 3 zeigt einen Magazinwechsler 9 mit einer linearen Anordnung der Magazine 8. Ein rastender Sicherungsbügel 19 sichert die Position 22 der Magazine 8 im Magazinwechsler 9. Ein Sensor erfasst, ob der Sicherungsbügel 19 geöffnet oder geschlossen ist. Nur bei geschlossem Sicherungsbügel 19 kann der Magazinwechsler 9 verschoben werden. Ein aufgebrachter Positionscode 21 auf dem Wechsler 9 bzw. auf der Linearführung 10 und ein Sensor erlauben die Erkennung der Magazinposition vor der Auswurfeinrichtung 5. Ein aufgebrachter Magazincode 20 und ein Sensor erlauben die Erkennung des Magazins 8 in der Magazinposition vor der Auswurfeinrichtung 5. Die Auswurfeinrichtung 5 kann nur vorgeschoben werden, wenn der Sicherheitsbügel 19 geschlossen ist, wenn die Magazinposition und der Magazintyp bekannt sind.

Anstatt der einsteckbaren Magazine 8 können auch Schläuche oder andere Zufuhrmechanismen verwendet werden, mit denen die Seeds 4 in den Magazinwechsler 9 transportiert werden.

Bei einer nicht dargestellten weiteren Ausführungsform des Magazinwechslers 9 sind die Magazine 8 nicht auf einer linearen Achse, sondern auf einer Kreisbahn geführt (Revolverwechsler).

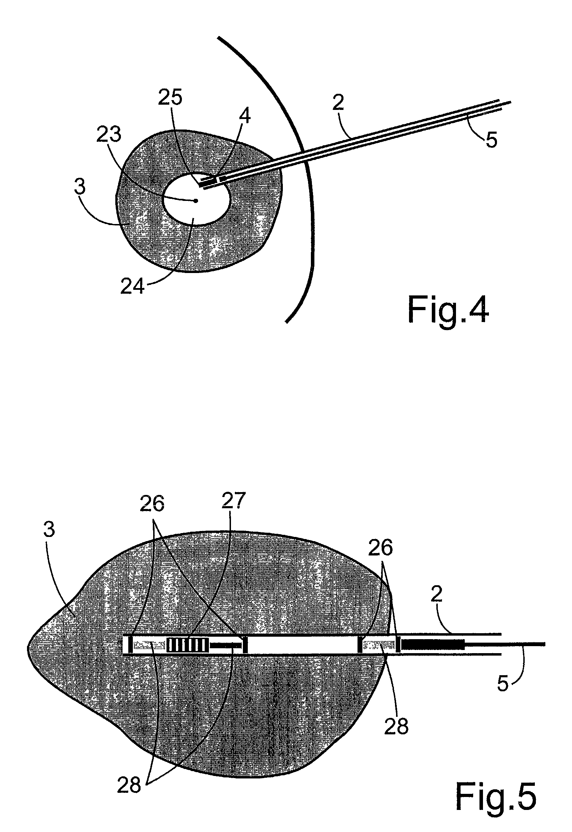

Fig. 4 zeigt den Auswurf von Einzelseeds 4 im Gewebe 3. Die Nadel 2 befindet sich im Tumorgewebe und wird zurückgezogen, bis die Seed-Position 25 für ein auszuwerfendes Seed 4 vor der Nadelspitze einen minimalen Abstand zu einer vorgeplanten kartesischen Position 23 relativ zu einem Lagereferenzkörper 13 (Fig. 1) besitzt und ein erlaubtes Toleranzintervall 24 nicht verlassen wird.

Die Position der Nadel 2 relativ zum Lagerefernzkörper 13 wird kontinuierlich über ein internes Meßsystem (Encoder), externes Meßsystem (z.B. optisches Navigationssystem) oder ein relatives Messystem (Ultraschall-Position der Nadelspitze im Tumorgewebe) erfasst.

Die Position der Auswurfeinrichtung 5 in der Nadel 2 wird kontinuierlich über ein internes Meßsystem (Encoder, Rast-Maßstab) erfasst.

Fig. 5 zeigt das Befüllen einer Nadel 2 mit Stoppern 26, aktiven Seeds 4 und Abstandshaltern 27 für zwei Gewebebereiche. Beide Gewebebereiche lassen sich mit nur einer Nadel 2 befüllen.

Zum Auswerfen wird die Auswurfeinrichtung 5 mechanisch relativ zum Lagereferenzkörper 13 fixiert. Anschließend wird die Nadelführung mit der Nadel 2 zurückgezogen.

Für das Einbringen von Einzelseeds 4 wird der Applikator 1 wie folgt verwendet:

Aus der Dosisplanung ist bekannt, welche Seed-Magazine 8 mit welchem Seed-Typ, welcher Dosis, Halbwertszeit und Anzahl aktuell für die Behandlung zur Verfügung stehen. Über die Dosisplanung werden diese Daten zur Aufstellung des aktuellen Behandlungsplans verwendet.

Die Hohlnadel 2 ist bereits bis zu ihrer maximalen vorgeplanten Eindringtiefe in das Gewebe 3 eingestochen. Die Lage (Position und Orientierung) der Nadelspitze relativ zu dem Lagereferenzkörper 13 ist bekannt. Die Lage (Position und Orientierung) des Tumorgewebes relativ zu dem Lagereferenzkörper 13 ist bekannt.

Der aktuelle Behandlungsplan ist bekannt. Der aktuelle Behandlungsplan ist eine Liste aller noch zu plazierenden Seeds 4 mit Angabe von Seed-Typ (z.B.: Jod, Palladium, Spacer, Stopper) sowie den x, y, und z-Koordinaten (Tiefe) der Position des Seeds 4 relativ zum Lagereferenzkörper 13. Es ist ein räumliches Toleranzintervall für jede Seed-Position bekannt, außerhalb dessen ein Seed 4 nicht abgelegt werden darf.

An die Nadel 2 wird der Applikator 1 über den Schnellverschluss 18 angeflanscht.

Die Nadel 2 wird jetzt soweit zurückgezogen, bis die Nadelspitze so positioniert ist, dass ein an der Nadelspitze ausgeworfenes Seed 4 anschließend einen minimalen Abstand zu einer im Behandlungsplan vorgesehenen Seed-Position 23 hat (Fig. 4). Ausgehend von der Seed-Position 23 wird jetzt das Magazin 8 des dazugehörigen Seed-Typs mit dem Magazinwechsler 9 vor der Auswurfeinrichtung 5 in Stellung gebracht. Mit der Auswurfeinrichtung 5 wird das Seed 4 aus dem Magazin 8 und durch die Hohlnadel 2 an der Nadelspitze in das Gewebe 3 geschoben.

Die Position 25 (Fig. 4), an der das Seed 4 tatsächlich positioniert wurde, wird an das Dosisplanungssystem zurückgemeldet. Optional ist zumindest die Position der Nadelspitze im Gewebe über ein bildgebendes System bekannt, um Lageveränderungen des Gewebes gegenüber dem Lagereferenzkörper zu erfassen und in Planung und Seedplazierung zu berücksichtigen.

Das Dosisplanungssystem kann daraufhin einen neuen aktuellen Behandlungsplan generieren. Dies geschieht unter Berücksichtigung aller bereits plazierten und noch zu plazierenden Seeds 4. Es geschieht ebenfalls auf der Basis der vor Ort noch in Magazinen 8 vorhanden Seeds 4.

Sind alle Seeds 4 eines Magazins 8 aufgebraucht, wird der Nutzer aufgefordert ein neues Seed-Magazin 8 in den Magazinwechsler 9 einzustecken.

Das Zurückziehen der Nadel 2, das Verschieben des Magazinwechslers 9 und das Auswerfen des Seeds 4 über die Auswurfeinrichtung 5 kann bei Bedarf motorisch angetrieben erfolgen, um Teilprozeße oder den gesamten Prozeß zu unterstützen.

Für das Vorladen von Hohlnadeln 2 wird der Applikator wie folgt verwendet:

Die Hohlnadel 2 ist bereits bis zu ihrer maximalen vorgeplanten Eindringtiefe in das Gewebe 3 eingestochen. Die Lage (Position und Orientierung) der Nadelspitze relativ zu dem Lagereferenzkörper 13 ist bekannt. Die Lage (Position und Orientierung) des Tumorgewebes relativ zu dem Lagereferenzkörper 13 ist bekannt.

Der aktuelle Behandlungsplan ist bekannt. Der aktuelle Behandlungsplan ist eine Liste aller noch zu plazierenden Seeds mit Angabe von Seed-Typ (z.B.: Jod, Palladium, Spacer, Stopper) sowie den x, y, und z-Koordinaten (Tiefe) der Position des Seeds 4 relativ zum Lagereferenzkörper 13. Es ist ein räumliches Toleranzintervall für jede Seed-Position bekannt, außerhalb dessen ein Seed nicht abgelegt werden darf.

An die Nadel 2 wird der Applikator 1 über den Schnellverschluss 18 angeflanscht.

Die Hohlnadel 2 wird jetzt mit Seeds 4 sowie Abstandshaltern 27 so befüllt, dass die Seeds 4 in der Nadel 2 anschließend einen minimalen Abstand zu einer im Behandlungsplan vorgesehenen Seed-Position 23 haben. Ausgehend von der jeweiligen Seed-Position wird dazu das Magazin 8 des dazugehörigen Seed-Typs (Stopper 26, Isotop 28 oder Abstandshalter 27) mit dem Magazinwechsler 9 vor der Auswurfeinrichtung 5 in Stellung gebracht. Es wird jetzt berechnet, an welcher Position in der Nadel 2 ein Seed 4 positioniert werden muss. Mit der Auswurfeinrichtung 5 wird das Seed 4 aus dem Magazin 8 an die Stelle in der Hohlnadel 2 geschoben, die für das Seed 4 oder den Abstandshalter 27 vorgesehen ist. Ist die Nadel 2 ausreichend befüllt, bleibt die Auswurfeinrichtung 5 in der Nadel 2 stehen, um die Seeds 4 in ihrer Position in der Nadel 2 zu stützen. Jetzt wird die Hohlnadel 2 langsam aus dem Gewebe 3 zurückgezogen. Die Auswurfeinrichtung 5 hält jedoch ihre Position relativ zum Lagereferenzkörper 13. Die Seeds 4 werden dabei im Gewebe 3 abgesetzt.

Die Position 25, an der das Seed 4 tatsächlich positioniert wurde, wird an das Dosisplanungssystem zurückgemeldet. Optional ist zumindest die Position der Nadelspitze im Gewebe über ein bildgebendes System bekannt, um Lageveränderungen des Gewebes gegenüber dem Lagereferenzkörper zu erfassen und in Planung und Seedplazierung zu berücksichtigen.

Das Dosisplanungssystem kann daraufhin einen neuen aktuellen Behandlungsplan generieren. Dies geschieht unter Berücksichtigung aller bereits plazierten und noch zu plazierenden Seeds. Es geschieht ebenfalls auf der Basis der vor Ort noch in Magazinen vorhanden Seeds.

Sind alle Seeds eines Magazins aufgebraucht, wird der Nutzer aufgefordert ein neues Seed-Magazin in den Magazinwechsler einzustecken.

Das Zurückziehen der Nadel, das Verschieben des Magazinwechslers und das Auswerfen des Seeds über die Auswurfeinrichtung 5 kann bei Bedarf motorisch angetrieben erfolgen, um Teilprozeße oder den gesamten Prozeß zu unterstützen.

Das Vorladen der Nadeln hat gegenüber den Einzelseeds den Verlust an Flexibilität bei der Planung und Behandlung zur Folge, wenn die Seeds nicht exakt wie vorgeplant in Tumorgewebe abgelegt werden oder abgelegt werden können. Wandern Seeds nach der Ablage oder kommt es zu einer fehlerhaften Bedienung sind die vorgeladenen Nadeln von Nachteil für die Behandlungsqualität.

Für das Erzeugen von Seed-Ketten und das extrakorporale Vorladen von Hohlnadeln kann der Applikator ebenfalls verwendet werden:

Das Verfahren nach der Erfindung kann auch dazu verwendet werden, um Nadeln extrakorporal zu befüllen, wenn die befüllten Nadeln später navigiert an vorgeplanten Positionen eingestochen und konventionell abgeladen werden.

Das Verfahren nach der Erfindung kann auch dazu verwendet werden, um Seeds in bio-absorbierbare Materialien abzulegen, aus denen sich Seed-Ketten mit bio-absorbierbaren Abstands- und Verbindungselementen herstellen lassen.

Das Vorladen der Nadeln hat gegenüber den Einzelseeds immer den Verlust an Flexibilität bei der Planung und Behandlung zur Folge, wenn die Seeds nicht exakt wie vorgeplant in Tumorgewebe abgelegt werden oder abgelegt werden können. Wandern Seeds nach der Ablage oder kommt es zu einer fehlerhaften Bedienung sind die vorgeladenen Nadeln von Nachteil für die Behandlungsqualität.

### Bezugszeichenliste

- 1: Applikator
- 2: Hohlnadel
- 3: Gewebe (Prostata)
- 4: Seed
- 5: Auswurfeinrichtung
- 6: Schlitten
- 7: Magazinwechsleraufnahme
- 8: Magazin
- 9: Magazinwechsler
- 10: Gleit-(Linear-)Führung
- 11: Fixierelement
- 12: Bremse
- 13: Lagereferenzkörper
- 14: Platte
- 15: Rektalsonde
- 16: Wechslerführung
- 17: Nadelaufnahme
- 18: Schnellverschluß
- 19: Sicherungsbügel
- 20: Magazincode
- 21: Rastpositionscode
- 22: Rastposition
- 23: Plan-Seed-Position
- 24: Toleranzintervall
- 25: Ist-Seed-Position
- 26: Stopper
- 27: Abstandshalter
- 28: Seed-Isotope

## Patentansprüche

1. Verfahren zum Positionieren von Strahlenquellen (Seeds), Abstandshaltern und Stoppern in Hohlnadeln,
**dadurch gekennzeichnet, dass** unterschiedliche Seeds (4), die nach Isotopentyp (Material, Dosis, Halbwertzeit) getrennt in Magazinen (8) enthalten sind, der Hohlnadel (2) derart zugeführt werden, dass das Wechseln der einzelnen Magazine (8) mittels eines Magazinwechslers (9) erfolgt, der in einer Wechslerführung (16) vertikal zur Injektionsrichtung der Seeds (4) geführt wird, wobei die Lage (Position und Orientierung) der Nadel (2) relativ zu einem Lagereferenzkörper (13) gemessen wird und eine optimale Seedablageposition (23) akustisch und/oder visuell und/oder taktil erfasst wird, wobei die Auswahl eines Magazins (8) und eines auszuwerfenden Seeds (4) aus dem Magazin (8) aus der aktuellen Position der Nadel (2) im Gewebe (3) und aus einem in Listenform mit Angabe des Seed-Typs und der x-y-z-Koordinaten relativ zu einem Lagereferenzkörper (13) erstellten Behandlungsplan für eine Dosisverteilung abgeleitet wird.

2. Verfahren nach Anspruch 1, **dadurch gekennzeichnet, dass** die lineare Bewegung der Nadel (2) und einer Magazinwechsleraufnahme (7) entkoppelt von der linearen Bewegung einer Auswurfeinrichtung (5) erfolgt.

3. Verfahren nach Anspruch 1 und 2, **dadurch gekennzeichnet, dass** die Orientierung des Magazinwechslers (9) relativ zu einem Lagereferenzkörper (13) fixiert und gleichzeitig eine lineare Rückwärtsbewegung des Magazinwechslers (9) erreicht wird.

4. Verfahren nach Anspruch 2, **dadurch gekennzeichnet, dass** die Lage der Auswurfeinrichtung (5) relativ zu einer Gleitführung (10) fixiert wird.

5. Verfahren nach Anspruch 2, **dadurch gekennzeichnet, dass** die Lage der Auswurfeinrichtung (5) relativ zu einem Lagereferenzkörper (13) fixiert wird.

6. Verfahren nach Anspruch 1, **dadurch gekennzeichnet, dass** die Lage des Magazinwechslers (9) relativ zu einem Lagereferenzkörper (13) fixiert wird.

7. Verfahren nach Anspruch 1, **dadurch gekennzeichnet, dass** die Position des Magazinwechslers (9) gemessen und der Magazintyp erfasst werden.

8. Verfahren nach Anspruch 1, **dadurch gekennzeichnet, dass** die Lage (Position und Orientierung) (25) der platzierten Seeds (4) an die Dosierungsplanung zurückgemeldet und daraufhin der Behandlungsplan aktualisiert wird.

9. Verfahren nach Anspruch 1, **dadurch gekennzeichnet, dass** in den Magazinen (8) neben Isotopen (28) mit unterschiedlicher Halbwertszeit auch Abstandshalter (27) und Stopper (26) aus bioabsorbierbaren Materialien zugeführt werden können.

10. Verfahren nach den Ansprüchen 1 bis 9, **dadurch gekennzeichnet, dass** anstatt der einsteckbaren Magazine (8) Schläuche oder andere Zufuhrmechanismen verwendet werden, mit denen die Seeds (4) in den Magazinwechsler (9) transportiert werden.

11. Verfahren nach den Ansprüchen 1 bis 10, **dadurch gekennzeichnet, dass** die Magazine (8) des Magazinwechslers (9) nicht auf einer linearen Achse, sondern auf einer Kreisbahn geführt werden (Revolverwechsler).

12. Applikator zum Positionieren von Strahlenquellen (Seeds), Abstandshaltern und Stoppern in Hohlnadeln und zum Auswerfen derselben aus Hohlnadeln,
**dadurch gekennzeichnet, dass** eine Magazinwechsleraufnahme (7) mit einem in einer Wechslerführung (16) angeordneten, vertikal zur Injektionsrichtung des Seeds führbaren Magazinwechsler (9) mit Magazinen (8), die verschiedene Seeds, jeweils getrennt nach Isotopentyp (Material, Dosis, Halbwertszeit), enthalten, eine Nadelaufnahme (17) für eine Hohlnadel (2) aufweist, an die über einen Schnellverschluss (18) die Magazinwechsleraufnahme (7) angeflanscht ist, wobei die Nadel (2) Seeds (4) aus einem der Magazine (8) aufnehmen und über eine Auswurfeinrichtung (5) wie Auswurfdraht oder Auswurfstilett ausgeben kann, wobei die Magazinwechsleraufnahme (7) mit einem Schlitten (6) mit einer Gleitführung (10) zur reproduzierbaren, genauen Positionierung der Nadel (2) verbunden ist.

13. Applikator nach Anspruch 12, **dadurch gekennzeichnet, dass** der Magazinwechsler (9) manuell oder motorisch antreibbar und schaltungsgesteuert verstellbar ist.

14. Applikator nach den Ansprüchen 12 und 13, **dadurch gekennzeichnet, dass** die Auswurfeinrichtung (5) manuell oder motorisch antreibbar und schaltungsgesteuert verstellbar und fixierbar ist.

15. Applikator nach den Ansprüchen 12 bis 14, **dadurch gekennzeichnet, dass** die Position der Magazinwechsleraufnahme (7) auf der Gleitführung (10) manuell oder motorisch antreibbar und schaltungsgesteuert verstellbar und fixierbar ist.

16. Applikator nach den Ansprüchen 12 bis 15, **dadurch gekennzeichnet, dass** die Lage des gesamten Applikators manuell oder motorisch antreibbar und schaltungsgesteuert verstellbar und fixierbar ist.
